(19) Europäisches Patentamt
European Patent Office
Office européen des brevets

(11) Publication number: **0 170 311**
**B1**

(12) **EUROPEAN PATENT SPECIFICATION**

(45) Date of publication of patent specification: **01.02.89**

(51) Int. Cl.[4]: **C 07 C 2/32,** C 07 C 2/36

(21) Application number: **85201048.7**

(22) Date of filing: **01.07.85**

(54) **Process for the dimerization of olefins.**

(30) Priority: **30.07.84 GB 8419407**
**09.11.84 GB 8428347**

(43) Date of publication of application:
**05.02.86 Bulletin 86/06**

(45) Publication of the grant of the patent:
**01.02.89 Bulletin 89/05**

(84) Designated Contracting States:
**BE DE FR GB NL**

(58) References cited:
**GB-A-1 131 146**
**GB-A-2 058 074**
**US-A-3 709 955**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Drent, Eit**
**Badhuisweg 3**
**NL-1031 CM Amsterdam (NL)**

(74) Representative: **Aalbers, Onno et al**
**P.O. Box 302**
**NL-2501 CH The Hague (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 170 311 B1

## Description

The present invention relates to a process for the dimerization in the liquid phase of an aliphatic mono-olefin having in the range of from 2 to 12 carbon atoms per molecule in the presence of a catalytic system containing palladium.

It is known that for dimerizing low molecular weight aliphatic mono-olefins a catalytic system can be used, which is formed by combining a palladium halide with an organoaluminum halide and preferably with a monodentate phosphine, arsine or stibine (cf. UK Patent Specification 1,153,519). It is also known to dimerize propene in the presence of a catalytic system formed by combining pentanedionatopalladium with ethylaluminum dichloride and an organic monodentate phosphine or phosphite (cf. Angew. Chem. Int. Ed. *14* (1975) 104—105.

Because of the presence of organoaluminum halides in the catalytic systems the above dimerization reactions should be carried out under strictly anhydrous conditions.

Further, it is known to dimerize ethene with a catalytic system formed by combining tetrakis(acetonitrile)palladium ditetrafluoroborate with a monodentate phosphine ligand (cf. J. Am. Chem. Soc. *103* (1981) 4627—4629 and *104* (1982) 3520—3522. Once again, this dimerization has to be carried out under strictly anhydrous conditions (cf. Transition metal catalyzed polymerisations: Alkenes and Dienes, Part A edited by R.P. Quirk, 1983, pp. 341—354).

From British Patent Specification No. 1,131,146 a process is known for the oligomerization and in particular to the dimerization and co-dimerization of monoalkenes, having in the range of from 2 to 12 carbon atoms per molecule, in the liquid phase, comprising contacting the monomer with a catalyst formed from a compound of a metal of Group VIII of the Periodic Table of the Elements, a chelate ligand comprising an organic compound containing as coordinating atoms at least two atoms of Group V of the Periodic Table of the Elements which are connected through a chain comprising 2 to 6 carbon atoms, and a compound acting as a Lewis acid under the process conditions.

However the actually disclosed process requires anhydrous and oxygen free reaction conditions and only uses nickel or cobalt compounds. This process shows in the Examples relatively low selectivities for the desired specific dimers, which causes additional laborious purification of the desired dimers.

Therefore there is still a need for a dimerization process, which combines high reaction rates and attractive high selectivities to dimers.

The present invention relates to a process for the dimerization in the liquid phase of an aliphatic mono-olefin having in the range of from 2 to 12 carbon atoms per molecule, comprising contacting the monomer with a catalyst formed from a compound of a metal of Group VIII of the Periodic Table of the Elements, a chelate ligand comprising an organic compound containing as coordinating atoms at least two atoms of Group V of the Periodic Table of the Elements, which are connected through a chain comprising 2 to 6 carbon atoms, characterized in that the dimerization is carried out in the presence of a catalytic system, formed by combining

(a) a palladium compound

(b) the chelate ligand and

(c) a compound containing an anion of an acid with the exception of hydrohalogenic acids,

in the presence of water, an alcohol or a carboxylic acid.

It is an advantage of the process of the invention that the catalytic systems used therein do not require anhydrous reaction conditions. In addition high reaction rates can be achieved whilst maintaining an attractive selectivity to dimers. The selectivity to dimers is defined as the molar percentage of dimers in the product formed.

Aliphatic mono-olefins having in the range of from 2 to 12 carbon atoms which can be used in the process according to the present invention are linear or branched alkenes or cycloalkenes, such as, for example, ethene, propene, 1-butene, 2-butene, the isomeric pentenes, hexenes, octenes and dodecenes, cyclopentene, cyclooctene and cyclododecene. Examples of other aliphatic mono-olefins are substituted alkenes such as allyl alcohol, acrylic acid and alkylesters of acrylic acid. The preferred olefins are ethene, propene and 1-butene.

The word "dimerization" as it is used herein, refers to the reaction of two identical olefins as well as the reaction of two different olefins.

According to the invention, both homogeneous and heterogeneous catalytic systems can be used. The use of homogeneous catalytic systems is preferred.

Palladium compounds which can be used in the process according to the invention therefore preferably comprise palladium compounds which are soluble in the reaction medium or form in situ soluble compounds therein. Examples of suitable palladium compounds are palladium nitrate, palladium sulphate, palladium halides and palladium carboxylates, preferably carboxylates of carboxylic acids having not more than 12 carbon atoms per molecule. Palladium carboxylates, in particular palladium acetate, are preferably used.

Further examples of suitable palladium compounds are palladium complexes such as bis(2,4-pentanedionato)palladium, bis(picolinato)palladium, tetrakis(triphenylphosphine)palladium, tetrakisacetonitrile palladium tetrafluoroborate, bis(tri-o-tolylphosphine)palladium acetate, bis(triphenylphosphine)palladium sulphate, palladium olefin complexes for instance di-μ-chlorodichlorobis(ethylene)dipalladium

($[Pd.C_2H_4.Cl_2]_2$), and di-μ-chlorodichlorobis(propylene)dipalladium ($[Pd.C_3H_6.Cl_2]_2$), and palladium-hydide complexes. The quantity of the palladium compound used may vary within wide ranges and is generally in the range between $10^{-6}$ and $10^{-1}$ mol palladium compound per mol olefin starting material. A range between $10^{-5}$ and $10^{-2}$ mol palladium compound is preferred.

The chelate ligand which may be used in the process according to the invention comprises an organic compound containing as coordinating atoms at least two atoms of Group 5a of the Periodic Table of the Elements which are connected through a chain comprising 2 to 6 carbon atoms. The Periodic Table of the Elements mentioned herein refers to that shown on the inside of the cover of "Handbook of Chemistry and Physics", 61st edition (1980—1981), CRC Press, Inc.

Suitable compounds may be compounds containing two nitrogen atoms which are connected through a chain comprising 2 carbon atoms such as 1,2-ethanediamine compounds for example N,N,N'-N'-tetramethyl-1,2-ethanediamine, N,N,N',N'-tetraethyl-2,2-ethanediamine and N,N,N',N'-tetraphenyl-1,2-ethanediamine, heterocyclic diamines for example 1,4-diphenylpiperazine, 1,4-dimethyl-1,4-dihydropyrazine and compounds containing in the molecule a group of the formula

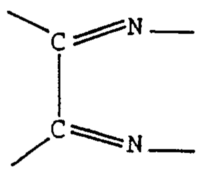
,

for example N,N'-1,2-ethanediylidenebisphenylamine, N,N'-1,2-ethanediylidenebis[4-chlorophenylamine], N,N'-1,2-ethanediylidenebis[4-methoxyphenylamine], N-substituted derivatives of 2-pyridinemethanimine, 2,2'-bipyridyl, 4,4'-dimethyl-2,2'-bipyridyl, 4,4'-dichloro-2,2'-bipyridyl, 4,4'-dimethoxy-2,2'-bipyridyl, 1,10-phenanthroline, 5-chloro-1,10-phenanthroline, 4,7-diphenyl-1,10-phenanthroline, 4,7-dimethyl-1,10-phenanthroline, 2,9-dichloro-1,10-phenanthroline, 1,10-phenanthroline-5-sulphonic acid, 4,7-diphenyl-1,10-phenanthrolinedisulphonic acid, and 3,5-cyclohexadiene-1,2-diimine.

Other suitable compounds may be compounds containing two phosphorus atoms, or two arsenic atoms or optionally a phosphorus atom or an arsenic atom in combination with a nitrogen atom which are connected through a chain comprising 2 carbon atoms such as for example 1,2-ethanediylbisdiphenylphosphine, 1,2-ethenediylbisphenylphosphine, 1,2-ethynediylbisdiphenylphosphine, 1,2-ethanediylbisdi-(trifluoromethyl)phosphine, 1,2-phenylenebisdiphenylphosphine, 1,2-tetrafluorocyclobutenediylbis-diphenylphosphine, 1,2-hexafluorocyclopentenediylbisdiphenylphosphine, 1,2-octafluorocyclohexenediylbisdiphenylphosphine, 1,4-diphenyl-1,4-diphosphacyclohexane, bis(o-diphenylphosphinophenyl)-phenylphosphine, tris(o-diphenylphosphinophenyl)phosphine, 1,2-phenylenebisdimethylarsine, 1,2-ethanediylbisdiphenylarsine, 1-dimethylamino-2-phenyldiethylphosphine, 8-dimethylarsinoquinoline, 10-methyl-5,10-dihydrophenarsazine, 1,2-tetrafluorophenylenebisdimethylarsine.

Further suitable compounds may be compounds containing at least two nitrogen atoms, phosphorus atoms or arsenic atoms connected through a chain comprising 3 to 5 carbon atoms such as for example N,N,N',N'-tetramethyl-1,3-propanediamine, N,N,N'N'-tetramethyl-1,4-butanediamine, 1,3-propanediylbisdiphenylphosphine, 1,4-butanediylbisdiphenylphosphine, bis(bis-3-dimethylarsinopropyl)arsine, tetrakis(3-dimethylarsinopropyl)o-phenylenediarsine.

The compounds preferably used in the catalytic system used in the process according to the invention are 1,10-phenanthroline and the derivatives thereof, 2,2'-bipyridyl and the derivatives thereof and bis-diphenylphosphine compounds in which the two phosphorus atoms are connected through a chain comprising 2 or 3 carbon atoms.

The quantity of chelate ligands used in the catalytic system is at least 0.1 mol ligand per gram atom palladium and preferably varies between 1 and 25 mol ligand per gram atom palladium.

The catalytic system used in the process of the invention is further formed by a compound containing an anion of an acid, with the exception of hydrohalogenic acids. The anion is preferably a non-coordinating anion, by which is meant that little or no covalent interaction takes place between the palladium and the anion (cf. UK Patent Application No. 2,058,074). Typical examples of such anions are $PF_6^-$, $SbF_6^-$, $BF_4^-$ and $ClO_4^-$.

The anion-containing compound may be a salt or a metal complex compound containing an anion of an acid or may be the acid itself. The acid preferably has a pKa of less than 3 and, more preferably, less than 2, measured in aqueous solution at a temperature of 18°C.

Preferred compounds are those containing anions of for example sulphonic acids and acids that can be formed, possibly in situ, by interacting a Lewis acid such as, for example, $BF_3$, $AsF_5$, $SbF_5$, $PF_5$, $TaF_5$ or $NbF_5$ with a Broensted acid such as, for example, a hydrohalogenic acid, in particular HF, fluorosulphonic acid, phosphoric acid or sulphuric acid. Specific examples of acids of the latter type are fluorosilicic acid, $HBF_4$, $HPF_6$ and $HSbF_6$. Examples of usable sulphonic acids are fluorosulphonic acid and chlorsulphonic acid and the hereinafter specified sulphonic acids.

A preferred group of compounds are compounds containing anions of acids having the general formula I

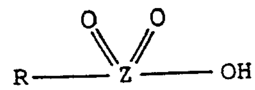

(I)

wherein Z represents sulphur or chlorine and, if Z is chlorine, R represents oxygen and, if Z is sulphur, R represents an OH group or an optionally substituted hydrocarbon group.

When the hereinbefore-stated anion-containing compounds are used in the process according to the invention, the anions of the compounds can be considered to be non-coordinating.

The optionally substituted hydrocarbon group represented by R is preferably an alkyl, aryl, aralkyl or alkaryl group having 1 to 30, in particular 1 to 14, carbon atoms. The hydrocarbon group may, for example, be substituted with the halogen atoms, in particular fluorine atoms. Examples of suitable acids of the general formula I are perchloric acid, sulphuric acid, 2-hydroxypropane-2-sulphonic acid, benzenesulphonic acid, 1- and 2-naphthalenesulphonic acid, p-toluenesulphonic acid and trifluoromethanesulphonic acid, the last two acids being the most preferred.

According to another embodiment of the present invention the catalytic system is formed by using a compound containing an anion of a carboxylic acid as component c). Examples of suitable carboxylic acids are formic acid, acetic acid, monochloroacetic acid, dichloroacetic acid, trichloroacetic acid and trifluoroacetic acid. Very good results have been obtained with the latter acid.

The anion-containing compound is preferably used in the form of the acid itself. However, under certain conditions it is possible to use salts containing an anion of an acid, which can be exchanged with the anion of the palladium compound used. For example, $AgBF_4$, $AgSbF_6$ or Ag-*p*-toluene sulphonate can be used when the palladium compound is palladium chloride or a palladium complex compound containing chloride anions.

The compound containing an anion of the acid is preferably used in a quantity in the range of from 0.01 to 150 and in particular 1 to 100 equivalents per gram atom palladium.

It will be appreciated that when the catalytic system applied in the process according to the invention is formed by combining in situ a palladium compound, a chelate ligand and a compound containing an anion of an acid, a palladium complex compound with catalytic activity may be formed in the reaction mixture. An example of such a compound is palladium bis(1,10 phenanthroline)diperchlorate or ditosylate. The use of such a palladium complex compound when prepared separately as catalytic system is within the scope of the present invention.

In the process of the invention the catalytic system is used in the presence of the protic solvents water, alcohol, or carboxylic acid. The alcohols or carboxylic acids may be aliphatic, cycloaliphatic or aromatic and may be substituted with one or more substituents for example alkoxy, cyano, ester groups or halogen atoms. The alcohols or carboxylic acids preferably contain not more than 20 carbon atoms per molecule. Examples of suitable alcohols are methanol, ethanol, propanol, isobutylalcohol, tert.butylalcohol, stearyl alcohol, benzyl alcohol, cyclohexanol, allyl alcohol, chlorocapryl alcohol, ethylene glycol, 1,2-propanediol, 1,4-butanediol, glycerol, polyethylene glycol, 1,6-hexanediol, phenol, cresol. Special preference is given to alcohols having 1 to 10 carbon atoms per molecule.

Examples of suitable carboxylic acid solvents are acetic acid, propionic acid, butyric acid, caproic acid, trimethylacetic acid, benzoic acid, caprylic acid, succinic acid, adipic acid and hydroxycaproic acid.

The amount of water, alcohol or carboxylic acid solvent used in the reaction mixture may be any amount that activates the catalytic system. Conveniently, such amounts can be used that water serves as co-solvent and the alcohol or carboxylic acid as solvent or co-solvent.

The process according to the invention is carried out in the liquid phase which may comprise a liquid mixture of the olefin, the catalytic system, reaction products, water, an alcohol or a carboxylic acid and preferably a solvent or co-solvent.

As stated hereinabove, water, alcohols or carboxylic acids can be used as solvent or co-solvent. Further examples of solvents and co-solvents are hydrocarbons, in particular aromatic hydrocarbons such as hexane, benzene or toluene, halogenated hydrocarbons such as chloroform, chlorobenzene or perfluoroalkanes, ketones such as acetone, diethyl ketone or methyl isobutyl ketone, ethers such as tetrahydrofuran, dimethylether of diethylene glycol (also referred to as "diglyme"), methyl t-butyl ether or 1,4-dioxane, sulphones such as dimethyl sulphone, methyl butyl sulphone, tetrahydrothiophene 1,1-dioxide (also referred to as "sulpholane") and sulphoxides such as dimethyl sulphoxide or diethyl sulphoxide.

The liquid phase may comprise a two-phase liquid system. For example when ethene or propene is dimerized using a diol as solvent, the dimerization product may form a separate layer which can be easily separated from diol solvent containing the catalytic system.

The process according to the present invention can be carried out at temperatures of up to 200°C and preferably in the range 20°C and 135°C. The pressure preferably lies between 1 and 100, in particular between 20 and 75, bar gauge.

The process according to the invention can be carried out batchwise, semi-continuously or continuously. The reaction time may vary in relation to the temperature used, between 0.5 and 20 hours.

The following Examples further illustrate the invention. The Examples 2—9 and Comparative Experiment A were carried out as described for Example 1 except for the catalytic system. The results are

presented in Table I.

Example 1

A 300 ml magnetically stirred Hastelloy C autoclave ("Hastelloy" is a trade mark) was charged with 50 ml methanol and a catalytic system formed by combining 0.1 mmol of palladium acetate, 0.15 mmol of 1.3-propanediylbisdiphenylphosphine and 2 mmol of p-toluenesulphonic acid. The autoclave was flushed with ethene, filled with ethene at a pressure of 40 bar, sealed and heated to a temperature as indicated in Table I. After a reaction time as indicated in Table I the contents of the autoclave were analyzed by gas/liquid chromatography.

The conversion of ethene to products (dimers, trimers, etc.) was calculated as mol of ethene per gram atom palladium per hour.

Example 2

The catalytic system was formed by combining 0.5 mmol palladium acetate, 1 mmol 1,10-phenanthroline and 10 mmol acetic acid.

Example 3

The catalytic system was formed by combining 0.5 mmol palladium acetate, 1 mmol 1,10-phenanthroline and 2 mmol monochloroacetic acid.

Example 4

The catalytic system was formed by combining 0.5 mmol palladium acetate, 1 mmol 1,10-phenanthroline and 2 mmol dichloroacetic acid.

Example 5

The catalytic system was formed by combining 0.5 mmol palladium acetate, 1 mmol 1,10-phenantroline and 22 mmol trifluoroacetic acid. The selectivities to trimers and tetramers were 25% and 2%, respectively.

Example 6

As catalytic system was added a composition of palladium complex compounds containing 0.1 mgram atom palladium. The composition was prepared as follows: Palladium acetate was dissolved in methanol and 3 mol of p-toluenesulphonic acid per gram atom palladium and 1.5 mol of 1,3-propanediylbisdiphenylphosphine per gram atom palladium were added. The resulting precipitate was isolated by filtration and washed with methanol.

Comparative Experiment A

As catalytic system was added a composition prepared analogously to the composition of Example 6 except that 3 mol of HCl (11 N) was used instead of 3 mol of p-toluenesulphonic acid.

Example 7

As catalytic system were added 0.1 mmol of palladium acetate, 2 mmol of 2,2'-bipyridyl and 1 mmol of p-toluenesulphonic acid.

Example 8

As catalytic system were added 0.1 mmol of palladium acetate, 2 mmol of 1,10-phenanthroline and 1 mmol of p-toluenesulphonic acid.

Example 9

As catalytic system was added-a composition prepared analogously to the composition of Example 6 except that 2 mol of 1,10-phenanthroline per gram atom palladium was used instead of 1.5 mol of 1,3-propanediylbisdiphenylphosphine.

TABLE I

| Comparative Experiment | Example No. | Temp, °C | Time, h | Conversion mol/gr.at Pd/h | Selectivity to dimer %mol |
|---|---|---|---|---|---|
| | 1 | 95 | 0.5 | 6000 | 98 |
| | 2 | 75 | 5 | 120 | |
| | 3 | 75 | 5 | 200 | |
| | 4 | 75 | 5 | 300 | - |
| | 5 | 75 | 0.16 | 5600 | 73 |
| | 6 | 95 | 0.5 | 6000 | 98 |
| | 7 | 75 | 0.5 | 8000 | 85 |
| | 8 | 90 | 1 | 5000 | 93 |
| | 9 | 95 | 2 | 2400 | 95 |
| A | | 115 | 5 | 70 | 100 |

Comparative Experiment A, not according to the invention, shows that a high conversion is not achieved in the presence of chloride anions.

Example 10

A 300 ml magnetically stirred Hastelloy C autoclave ("Hastelloy" is a trade mark) was charged with 50 ml diglyme, 5 ml water and as catalytic system 0.1 mmol of palladium acetate, 2 mmol of 2,2'-bipyridyl and 2 mmol of p-toluenesulphonic acid. The autoclave was flushed with ethene, filled with ethene at a pressure of 40 bar, sealed and heated to 90°C. After a reaction time of 1 hour the contents of the autoclave were analyzed by gas/liquid chromatography.

The converson of ethene to dimers and trimers was calculated as 2800 mol of ethene per gram atom palladium per hour. Butenes were present in an amount of 93 %mol in the product.

Comparative Experiment B

The experiment of Example 10 was repeated except that no water was present and that the autoclave was heated to 120°C during 5 hours. On analysis only traces of butenes were found, showing that the catalytic system effects dimerization in the presence of water as protic compound.

Example 11

A 300 ml magnetically stirred Hastelloy C autoclave ("Hastelloy" is a trade mark) was charged with 35 ml phenol and as catalytic system 0.1 mmol of palladium acetate, 4 mmol of 2,2'-bipyridyl and 4 mmol of p-toluenesulphonic acid. The autoclave was flushed with ethene and filled with ethene at a pressure of 40 bar, sealed and heated to 80°C. After a reaction time of 5 hours the contents of the autoclave were analyzed by gas/liquid chromatography.

The conversion of ethene to dimers and trimers was calculated as 800 mol of ethene per gram atom palladium per hour. Butenes were present in an amount of 90 %mol in the product.

Example 12

A 300 ml magnetically stirred Hastelloy C autoclave ("Hastelloy" is a trade mark) was charged with 50 ml acetic acid and as catalytic system 0.1 mmol of palladium acetate, 0.15 mmol of 1,3-propanediylbis-diphenylphosphine and 2 mol of trifluoromethanesulfonic acid. The autoclave was flushed with ethene and filled with ethene at a pressure of 40 bar, sealed and heated to 135°C. After a reaction time of 5 hours the contents of the autoclave were analyzed by gas/liquid chromatography.

The conversion of ethene to dimer products (butene 40 %mol and sec.butyl acetate 60 %mol) was calculated as 500 mol of ethene per gram atom palladium per hour. The selectivity to dimer products was 100 %mol.

Examples 13—19

A 300 ml magnetically stirred Hastelloy C autoclave ("Hastelloy" is a trade mark) was charged with a solvent and as catalytic system palladium acetate, a bidentate ligand and an acid with a pKa $< 2$. The autoclave was flushed with propene, filled with propene in the liquid phase at 30 bar in a quantity of 50 ml, sealed and heated to a certain temperature. After a certain reaction time the contents of the autoclave were analyzed by gas/liquid chromatography.

The conversion of propene to products was calculated as mol of propene per gram atom palladium per hour. The selectivity to the dimers is given as %mol of dimer in the products formed. The linearity of the dimer products is determined by NMR and is given as %mol of linear hexenes in the dimer products.

Data and results of the examples 13—19 carried out according to the above are mentioned in Table II.

TABLE II

| Example No. | Palladium acetate mmol | Bidentate ligand mmol | Acid pKa <2 mmol | Solvent ml | Reaction time h | Temp. °C | Conversion m/g at Pd/h | Selectivity %mol | Linearity %mol |
|---|---|---|---|---|---|---|---|---|---|
| 13 | 0.2 | 2,2'bipyridyl (4) | p-toluenesul-phonic acid (6) | methanol (50) | 2 | 85 | 1050 | 98 | 65 |
| 14 | 0.5 | 1,10-phenan-throline (1) | p-toluenesul-phonic acid (1.5) | butanediol -(1.4) (50) | 1 | 75 | 1250 | 94 | 60 |
| 15 | 0.5 | 2,2'bipyridyl (1) | p-toluenesul-phonic acid (1.5) | ethylene glycol (50) | 1 | 75 | 1100 | 95 | 58 |
| 16 | 0.5 | 1,10-phenan-throline (1) | sulphuric acid (1.5) | diethylene glycol (50) | 1 | 70 | 1050 | >95 | 60 |
| 17 | 0.5 | 1,10-phenan-throline (1) | $HBF_4$ (3) | diethylene glycol (50) | 1/4 | 70 | 4000 | >95 | 59 |
| 18 | 0.5 | 4,7-diphenyl-1,10-phenan-throline (1) | trifluoro-methane-sulphonic acid (1.5) | ethylene glycol (50) | 1/2 | 75 | 1800 | >95 | 60 |
| 19 | 0.5 | 5-chloro-1,10-phenanthroline (1) | naphtalene-sulphonic acid (2) | diethylene glycol (50) | 1 | 75 | 930 | >95 | 59 |

Example 20

A 300 ml magnetically stirred Hastelloy C autoclave ("Hastelloy" is a trade mark) was charged with 50 ml 1,4-butanediol and as catalytic system 0.5 mmol of palladium acetate, 1 mmol of 1,10-phenanthroline and 1.5 mmol p-toluenesulphonic acid. The autoclave was flushed with 1-butene and filled with 1-butene in the liquid phase at 30 bar in a quantity of 60 ml and heated to 75°C. After a reaction time of 5 hours the contents of the autoclave were analyzed by gas/liquid chromatography.

The conversion of 1-butene to dimers was calculated as 30 mol of 1-butene per gram atom palladium per hour. Dimers were the only products.

Example 21

A 300 ml magnetically stirred Hastelloy C autoclave ("Hastelloy" is a trade mark) was charged with 50 ml methanol and as catalytic system 1 mmol of palladium acetate, 2 mmol of 2,2'-bipyridyl and 2.5 mmol p-toluenesulphonic acid. The autoclave was flushed with propene and filled with propene and 1-butene in the liquid phase at 30 bar in a quantity of 50 ml and 60 ml, respectively, and heated to 65°C.

The conversion to dimer product was calculated as 800 mol of propene and butene per gram atom palladium per hour. The selectivity to dimers was 100 %mol. The composition of the dimer products was hexenes 69 %mol, heptenes 28 %mol and octenes 3 %mol.

**Claims**

1. A process for the dimerization in the liquid phase of an aliphatic mono-olefin having in the range of from 2 to 12 carbon atoms per molecule, comprising contacting the monomer with a catalyst formed from a compound of a metal of Group VIII of the Periodic Table of the Elements, a chelate ligand comprising an organic compound containing as coordinating atoms at least two atoms of Group V of the Periodic Table of the Elements, which are connected through a chain comprising 2 to 6 carbon atoms, characterized in that the dimerization is carried out in the presence of a catalytic system, formed by combining:

a) a palladium compound,

b) the chelate ligand and

c) a compound containing an anion of an acid, with the exception of hydrohalogenic acids,

in the presence of water, an alcohol or a carboxylic acid.

2. A process as claimed in claim 1, characterized in that the acid mentioned in c) has a pKa of less than 3, measured in aqueous solution at a temperature of 18°C.

3. A process as claimed in claim 2, characterized in that the acid has a pKa of less than 2.

4. A process as claimed in any one of the preceding claims, characterized in that as anion a non-coordinating anion is used.

5. A process as claimed in any one of the preceding claims, characterized in that the compound containing an anion of an acid contains the anion of an acid of the general formula I

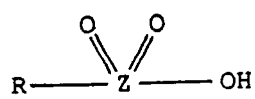

(I)

wherein Z represents sulphur or chlorine and, if Z is chlorine, R represents oxygen and, if Z is sulphur, R represents an OH group or an optionally substituted hydrocarbon group.

6. A process as claimed in claim 5, characterized in that the hydrocarbon group R is an alkyl, aryl, aralkyl or alkaryl group having 1 to 30 carbon atoms.

7. A process as claimed in claim 5 or 6, characterized in that the anion of an acid of the general formula I is the anion of p-toluenesulphonic acid.

8. A process as claimed in any one of claims 1 to 4, characterized in that the acid mentioned in c) is trifluoroacetic acid.

9. A process as claimed in any one of the preceding claims, characterized in that the compound containing an anion of an acid is used in the form of the acid itself.

10. A process as claimed in any one of the preceding claims, characterized in that the chelate ligand comprises a compound containing as coordinating atoms 2 nitrogen atoms connected through a chain comprising 2 carbon atoms.

11. A process as claimed in claim 10, characterized in that the chelate ligand comprises a compound containing in the molecule a group of the formula

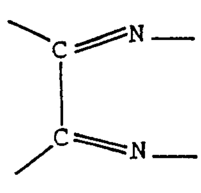

12. A process as claimed in claim 10 or 11, characterized in that the chelate ligand comprises 1,10-phenanthroline or a derivative thereof.

13. A process as claimed in claim 10 or 11, characterized in that the chelate ligand comprises 2,2'-bipyridyl or a derivative thereof.

14. A process as claimed in any one of claims 1 to 9, characterized in that the chelate ligand comprises a compound containing as coordinating atoms 2 phosphorus atoms connected through a chain comprising 2 or 3 carbon atoms.

15. A process as claimed in claim 14, characterized in that the chelate ligand comprises bisdiphenylphosphine compounds in which the phenyl groups are optionally substituted.

16. A process as claimed in claim 14 or 15, characterized in that the chelate ligands comprises 1,3-propanediylbisdiphenylphosphine.

17. A process as claimed in any one of the preceding claims, characterized in that the quantity of the chelate ligand varies between 1 and 25 mol ligand per gram atom palladium.

18. A process as claimed in any one of the preceding claims, characterized in that the compound containing an anion of an acid is used in a quantity in the range of from 1 to 100 equivalents per gram atom palladium.

19. A process as claimed in any one of the preceding claims, characterized in that water is used as a co-solvent.

20. A process as claimed in any one of claims 1 to 18, characterized in that an alcohol is used as solvent or co-solvent.

**Patentansprüche**

1. Verfahren zur Dimerisierung eines aliphatischen Mono-olefins mit 2 bis 12 Kohlenstoffatomen im Molekül in flüssiger Phase, indem das Monomere mit einem Katalysator aus einer Verbindung eines Metalls der VIII. Gruppe des Periodensystems und einem Chelat-Liganden enthaltend eine organische Verbindung mit einem Korordinationsatom an zumindest 2 Atomen der V. Gruppe des Periodensystems, die über eine Kette enthaltend 2 bis 6 Kohlenstoffatome miteinander verbunden sind, in Berührung gebracht wird, dadurch gekennzeichnet, daß die Dimerisierung ausgeführt wird in Gegenwart eines Katalysatorsystems aus
a) einer palladiumverbindung,
b) dem Chelat-Liganden und
c) einer Verbindung enthaltend ein Säureanion — mit Ausnahme von Halogenwasserstoffsäuren —
in Gegenwart von Wasser, einem Alkohol oder einer Carbonsäure.

2. Verfahren nach Anspruch 1, dadurch gekennzeichnet, daß die unter c) genannte Säure einen pKa-Wert von $< 3$ besitzt, bestimmt in wässriger Lösung bei 18°C.

3. Verfahren nach Anspruch 2, dadurch gekennzeichnet, daß die Säure einen pKa-Wert von $< 2$ hat.

4. Verfahren nach einem der vorhergehenden Ansprüche, dadurch gekennzeichnet, daß das Anion ein nicht-koordinatives Anion ist.

5. Verfahren nach einem der vorhergehenden Ansprüche, dadurch gekennzeichnet, daß die ein Säureanion enthaltende Verbindung ein Anion einer Säure der allgemeinen Formel I

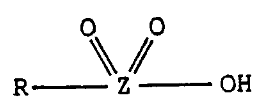

(I)

enthält, worin Z Schwefel oder Chlor ist und wenn Z Chlor ist, R Saurestoff bedeutet und wenn Z Schwefel ist, R eine OH-Gruppe oder eine gegebenenfalls substituierte Kohlenwasserstoffgruppe bedeutet.

6. Verfahren nach Anspruch 5, dadurch gekennzeichnet, daß die Kohlenwasserstoffgruppe R eine Alkyl-, Aryl-, Aralkyl- oder Alkarylgruppe mit 1 bis 30 Kohlenstoffatomen ist.

7. Verfahren nach Anspruch 5 oder 6, dadurch gekennzeichnet, daß das Anion der Säure der Formel I ein solches der p-Toluol-sulfonsäure ist.

8. Verfahren nach einem der Ansprüche 1 bis 4, dadurch gekennzeichnet, daß die unter c) genannte Säure Trifluoressigsäure ist.

9. Verfahren nach einem der vorhergehenden Ansprüche, dadurch gekennzeichnet, daß die ein Säureanion enthaltende Verbindung in Form der Säure angewandt wird.

10. Verfahren nach einem der vorhergehenden Ansprüche, dadurch gekennzeichnet, daß der Chelat-Ligand eine Verbindung, enthaltend als Koordinationsatome 2 Stickstoffatome aufweist, die über eine Kette von 2 Kohlenstoffatomen verbunden sind.

11. Verfahren nach Anspruch 10, dadurch gekennzeichnet, daß der Chelat-Ligand eine Verbindung enthaltend die Gruppierung

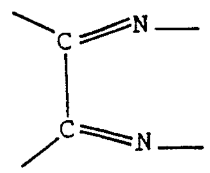

ist.

12. Verfahren nach Anspruch 10 oder 11, dadurch gekennzeichnet, daß der Chelat-Ligand 1,10-Phenanthrolin oder dessen Derivat enthält.

13. Verfahren nach Anspruch 10 oder 11, dadurch gekennzeichnet, daß der Chelat-Ligand 2,2'-Bisphenyl oder dessen Derivat enthält.

14. Verfahren nach einem der Ansprüche 1 bis 9, dadurch gekennzeichnet, daß der Chelat-Ligand eine Verbindung 2 Phosphoratomen als Korordinationsatome aufweist, die über eine Kette mit 2 oder 3 Kohlenstoffatomen verbunden sind.

15. Verfahren nach Anspruch 14, dadurch gekennzeichnet, daß der Chelat-Ligand ein Bisdiphenylphosphin ist, worin die Phenylgruppen gegebenenfalls substituiert sein können.

16. Verfahren nach Anspruch 14 oder 15, dadurch gekennzeichnet, daß der Chelat-Ligand 1,3-Propandiylbisdiphenylphosphin ist.

17. Verfahren nach einem der vorhergehenden Ansprüche, dadurch gekennzeichnet, daß der Chelat-Ligand in einer Menge von 1 bis 12 mol/g-Atom Palladium angewendet wird.

18. Verfahren nach einem der vorhergehenden Ansprüche, dadurch gekennzeichnet, daß die ein Säureanion enthaltend Verbindung in einer Menge von 1 bis 100 Äquivalent/g-Atom Palladium verwendet wird.

19. Verfahren nach einem der vorhergehenden Ansprüche, dadurch gekennzeichnet, daß als Co-Lösungsmittel Wasser verwendet wird.

20. Verfahren nach Anspruch 1 bis 18, dadurch gekennzeichnet, daß als Lösungsmittel oder Co-Lösungsmittel ein Alkohol verwendet wird.

**Revendications**

1. Procédé de dimérisation en phase liquide d'une monooléfine aliphatique comportant de 2 à 12 atomes de carbone par molécule, ce procédé comprenant la mise en contact du monomère avec un catalyseur formé à partir d'un composé d'un métal du groupe VIII de la Classification Périodique des Eléments, d'un coordinat chélate comprenant un composé organique contenant, comme atomes de coordination, au moins 2 atomes du groupe V de la Classification Périodique des Eléments, reliés par une chaîne comprenant 2 à 6 atomes de carbone, caractérisé en ce que la dimérisation est réalisée en présence d'un système catalytique formé par la combinaison
(a) d'un composé du palladium
(b) du coordinat chélate et
(c) d'un composé contenant un anion d'un acide, à l'exception des acides halogènhydriques,
en présence d'eau, d'un alcool ou d'un acide carboxylique.

2. Procédé selon la revendication 1, caractérisé en ce que l'acide indiqué en c) a un pKa inférieur à 3, mesuré en solution aqueuse à une température de 18°C.

3. Procédé selon la revendication 2, caractérisé en ce que l'acide a un pKa inférieur à 2.

4. Procédé selon l'une quelconque des revendications précédentes, caractérisé en ce que l'on utilise, comme anion, un anion non coordinant.

5. Procédé selon l'une quelconque des revendications précédentes, caractérisé en ce que le composé contenant un anion d'un acide contient l'anion d'un acide de formule générale I

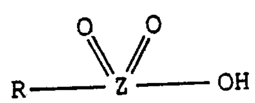

(I)

dans laquelle Z représente un soufre ou un chlore et, si Z est un chlore, R représente un oxygène et, si Z est un soufre, R représente un groupe OH ou un groupe hydrocarboné éventuellement substitué.

6. Procédé selon la revendication 5, caractérisé en ce que le groupe hydrocarboné R est un groupe alkyle, aryle, arylalkyle ou alkylaryle comportant 1 à 30 atomes de carbone.

7. Procédé selon la revendication 5 ou 6, caractérisé en ce que l'anion d'un acide de formule générale I est l'anion de l'acide p-toluène-sulfonique.

8. Procédé selon l'une quelconque des revendications 1 à 4, caractérisé en ce que l'acide indiqué en c) est l'acide trifluoroacétique.

9. Procédé selon l'une quelconque des revendications précédentes, caractérisé en ce que le composé contenant un anion d'un acide est employé sous forme de l'acide lui-même.

10. Procédé selon l'une quelconque des revendications précédentes, caractérisé en ce que le coordinat chélate comprend un composé contenant, comme atomes de coordination, 2 atomes d'azote reliés par une chaîne comprenant 2 atomes de carbone.

11. Procédé selon la revendication 10, caractérisé en ce que le coordinat chélate comprend un composé contenant, dans la molécule, un groupe de formule

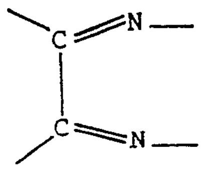

12. Procédé selon la revendication 10 ou 11, caractérisé en ce que le coordinat chélate comprend la 1,10-phénanthroline ou un de ses dérivés.

13. Procédé selon la revendication 10 ou 11, caractérisé en ce que le coordinat chélate comprend le 2,2'-bipyridyle ou un de ses dérivés.

14. Procédé selon l'une quelconque des revendications 1 à 9, caractérisé en ce que le coordinat chélate comprend un composé contenant, comme atomes de coordination, 2 atomes de phosphore reliés par une chaîne comprenant 2 ou 3 atomes de carbone.

15. Procédé selon la revendication 14, caractérisé en ce que le coordinat chélate comprend des composés bisdiphénylphosphines dans lesquels les groupes phényle sont éventuellement substitués.

16. Procédé selon la revendication 14 ou 15, caractérisé en ce que le coordinat chélate comprend la 1,3-propane-diylbisdiphénylphosphine.

17. Procédé selon l'une quelconque des revendications précédentes, caractérisé en ce que la quantité du coordinat chélate varie entre 1 et 25 moles de coordinat par atome-gramme de palladium.

18. Procédé selon l'une quelconque des revendications précédentes, caractérisé en ce que le composé contenant un anion d'un acide est employé en une quantité dans l'intervalle de 1 à 100 équivalents par atome-gramme de palladium.

19. Procédé selon l'une quelconque des revendications précédentes, caractérisé en ce que l'on utilise de l'eau comme co-solvant.

20. Procédé selon l'une quelconque des revendications 1 à 18, caractérisé en ce que l'on utilise un alcool comme solvant ou co-solvant.